# EUROPEAN PATENT APPLICATION

(11) **EP 1 595 504 A1**
(43) Date of publication of application: **16.11.2005**
(21) Application number: 05010023.9
(22) Date of filing: 09.05.2005
(51) Int. Cl.: A61B 17/00

(54) **Cardiovascular defect patch**

(30) Priority: 12.05.2004 US 845439
(71) Applicant: Medtronic Vascular, Inc., Santa Rosa, California 95403 (US)
(72) Inventor: Nayak, Asha, Menlo Park, CA 94025 (US); Saint, Sean, San Diego, CA 92109 (US); Guinan, Terry, Crinkle Birr, Co. Offaly (IE); Thornton, Ronan M., Co. Galway (IE); Hanley, Patrick, Barna, Co. Galway (IE); Mulvihill, Hilda, Co. Limerick (IE)
(74) Representative: Zimmermann, Gerd Heinrich

(57) **Abstract**

The present invention provides a defect patch device and method that patches a defect in the heart or other cardiovascular tissue. One aspect provides a PFO closure device and method that patches a PFO in the right atrium without the device extending through the PFO into the left atrium. The patch device includes a patch and a heart tissue engaging member for attaching the device over the defect. A deployment device and method includes a device positioner to advance the device out of a catheter and to position the device over the defect to attach the device to the tissue around the defect. The positioner may include a device expander or opener that opens the device for deployment.

## Description

### Field of the Invention:

The invention relates to a device and method for closing or patching a hole or defect in a cardiovascular organ, especially a defect in a heart such as a defect in the septum.

### Background of the Invention:

A variety of defects can occur in the wall of a heart or major vessels that may be treated by repair, closure or patching. Such defects may include, for example, holes or other defects in the atrial septum or ventricular septum. (ASD or VSD), ventricular aneurysms, patent foramen ovales, aortic dissections, ventricular free wall rupture and various vascular ruptures or holes.

A more frequently occurring of such defects is a patent foramen ovale ("PFO"). A PFO is an opening between the atria of the heart that results from an incomplete closure of the atrial septum shortly after birth. A thin tissue flap on the left atrial side of the septum, which represents an embryological remnant of the septum primum, forms the valve of the fossa ovalis. After birth, normally the left atrial pressure exceeds the right atrial pressure and forces the valve against the limbus, thus typically achieving physiological closure. The PFO defect occurs when the closure is not complete. The PFO works like a flap valve, with overlapping flaps that open up when a patient creates more pressure inside their chest, which can occur, e.g., when people cough, sneeze, or strain at stool. If the pressure is enough to open the defect, blood can flow between the atria. In particular, if blood flows from the right atrium to left atrium and if there are clots or other debris present in the blood at the site of the defect or crossing it, embolisms, clots, or obstructions can form, e.g., lodging in the brain causing a stroke, or in the heart causing a heart attack.

Currently there are devices for closing a PFO that comprise a double umbrella that with a connector extending through the PFO. The opposing umbrellas sandwich the PFO from within opposing atria whereby the tissue grows around the device to close the defect in the atrial septum. A potential problem with this device is that it leaves hardware in the left atrium, which could cause thrombosis, embolism or perforation of critical structures if for example, the device breaks off in the left atrium. Furthermore, anything passing through the PFO may keep the PFO open and may widen it with respect to its native geometry.

Accordingly, it would be desirable to provide a device for treating, closing, repairing or patching cardiac and vascular defects.

It would also be desirable to provide a PFO closure patch device that can be deployed in a single chamber of the heart without extending through the PFO into the opposite atrium. It would also be desirable to provide a PFO closure device that reduces the possibility of clotting or device breakage in the left atrium. Therefore, it is an object of the present invention to overcome the above mentioned disadvantages of the prior art at least partially.

### Summary of the Invention:

This object is solved by a tissue defect patch device according to claim 1, 14 and 15 as well as by a method for patching a heart tissue defect according to claim 16 and a system for patching a defect in heart tissue according to claim 24. Further details, aspects, advantages and features of the present invention are apparent from the dependent claims, the description and the accompanying drawings. The present invention provides a device for patching cardiac and vascular defects. One aspect according to the present invention provides a PFO closure device that patches the PFO in the right atrium without the device extending through the PFO and further leaving hardware in the left atrium.

An embodiment of the invention comprises a patch formed of a material such as a woven Dacron, ePTFE, or metal that can be placed over a tissue defect area. Where the device is for patching a PFO it is configured to be delivered in the right atrium and placed over the PFO. The device further comprises at least one engaging or attachment element for attaching the patch to the wall of the right atrium over the defect.

In one variation of the embodiment, the device includes a spring support member supporting the patch and biasing the device toward a tissue or wall engaging position whereby the attachment element engages the wall of the heart. The spring support member may be formed of a shape memory alloy such as Nitinol.

In one embodiment, the device is in the form of an umbrella-like structure with spoke like spring members coupled to the patch material. In one variation of the embodiment, the device includes barbs that engage the wall of the right atrium. During deployment, the spokes may be hyper-extended to open the device and place it over the tissue defect area, then released whereby the spring of the spokes biases the umbrella in a wall engaging position with the barbs piercing the atrial wall to hold the patch in position over the tissue defect.

### Brief Description of the Drawings:

Figure 1 is a schematic front view of a heart with a PFO.

Figure 2A is a schematic front view of a portion of heart with a PFO and a device according to the invention placed over the PFO.

Figure 2B is a schematic side view, partial breakaway of a heart showing the implant from within the right atrium and device of Figure 2A.

Figure 2C is a cutaway detailed view of the distal side of the device of Figure 2A.

Figure 3A is perspective view of a PFO closure device according to the invention.

Figure 3B is a side view of the patch of Figure 3A

Figure 4 is a schematic side view of a PFO device in an embodiment of a delivery catheter according to the invention.

Figure 5 is a schematic side view of the PFO device and the delivery catheter of Figure 4 with the device advanced out of the catheter in its relaxed position.

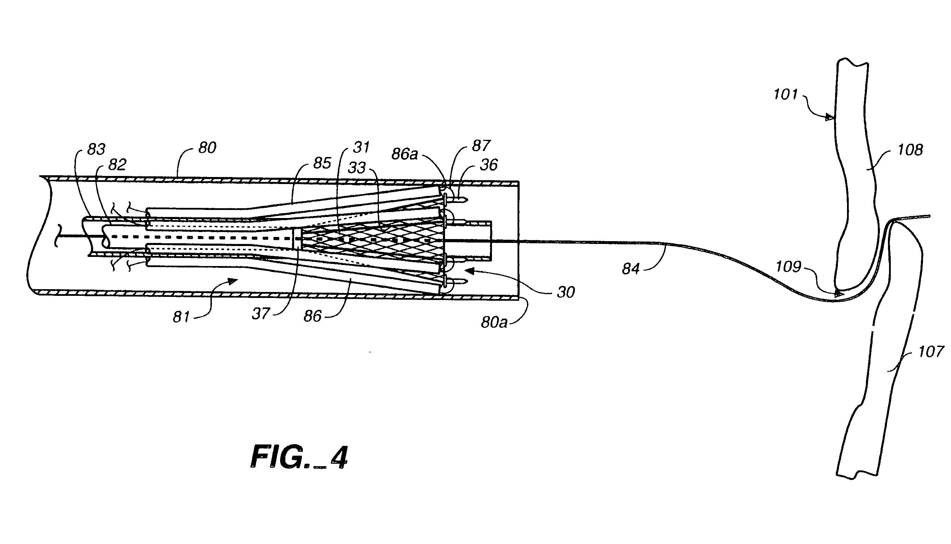

6 is a schematic side view of the PFO device and the delivery catheter of Figure 4 with the device advanced out of the catheter in a hyper-extended position for placement over a PFO.

7 is a perspective view of the PFO device and catheter of Figures 4-6.

8 is a perspective view of an alternative embodiment of a PFO device and delivery catheter according to the invention.

9 is a perspective view of an alternative embodiment of a PFO device and delivery catheter according to the invention.

10 is a side view of an embodiment of a PFO device according to the invention with the device in a relaxed position.

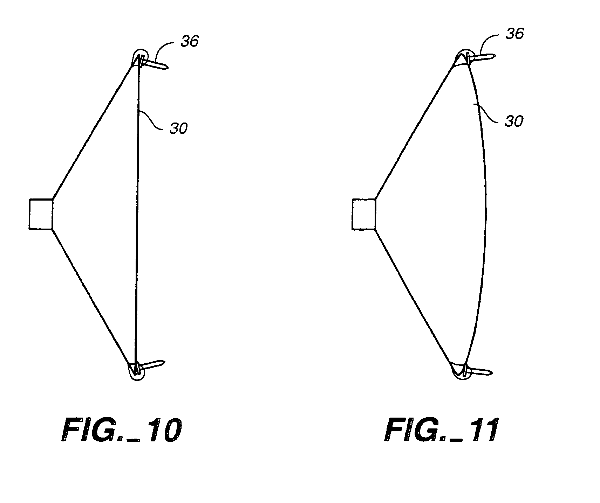

11 is a side view of the PFO device of Figure 10 with the device in a hyper-extended position.

12 is a schematic side cross section of a variation of the delivery system of Figures 4-7.

13 illustrates a side view of a variation of the delivery system of figures 4-7.

14A is a side view of another embodiment of a PFO closure device according to the invention in a predeployment configuration.

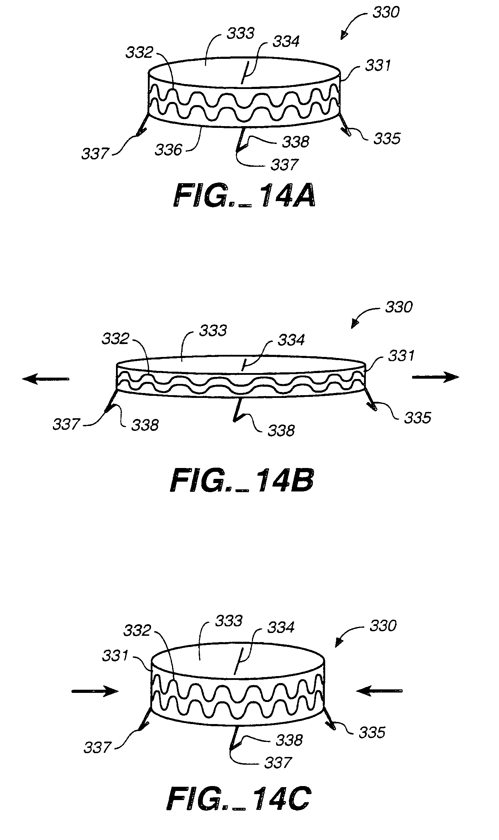

14B is a side view of the device of Figure 14A in an expanded configuration.

14C is a side view of the device of Figure 14B after it is released and recoils towards its predeployment configuration.

15 is a side view of the PFO closure device of Figure 14A as it is being placed over a PFO.

16 is a side view of the PFO closure device of Figure 14A in place over a PFO.

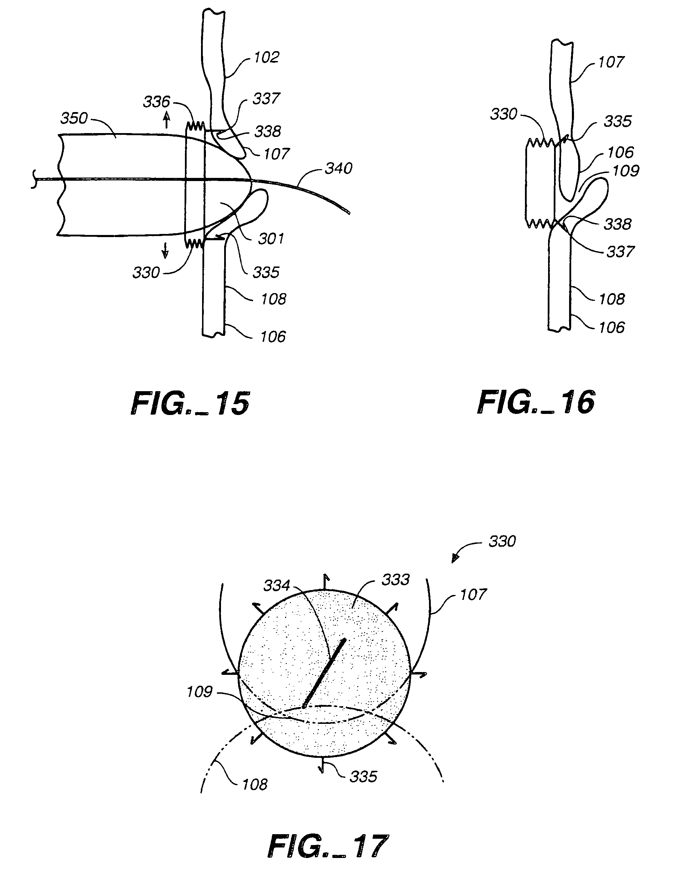

Figure 17 is a top view of the deployed PFO closure device as illustrated in Figure 16.

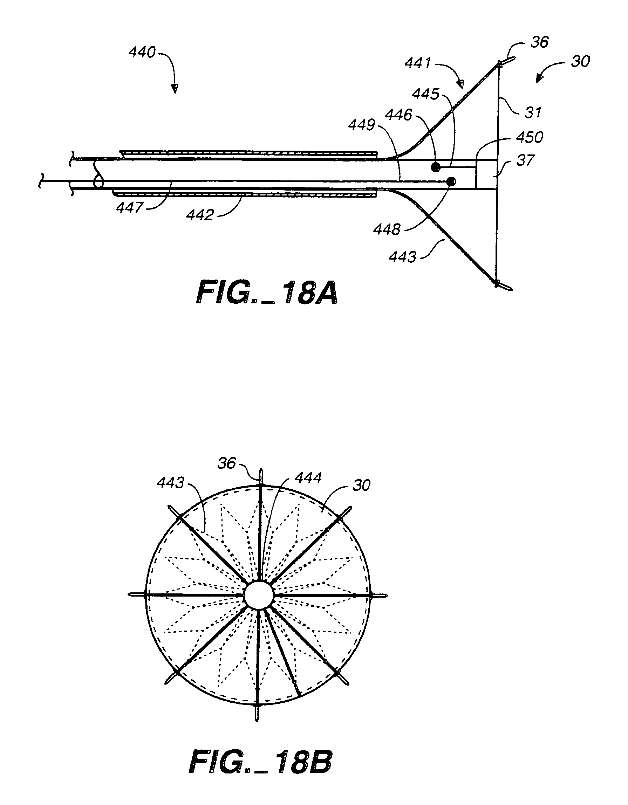

Figure 18A is a side cross section of an alternative PFO closure device and delivery system.

Figure 18B is front view of the delivery system as illustrated in Figure 18A.

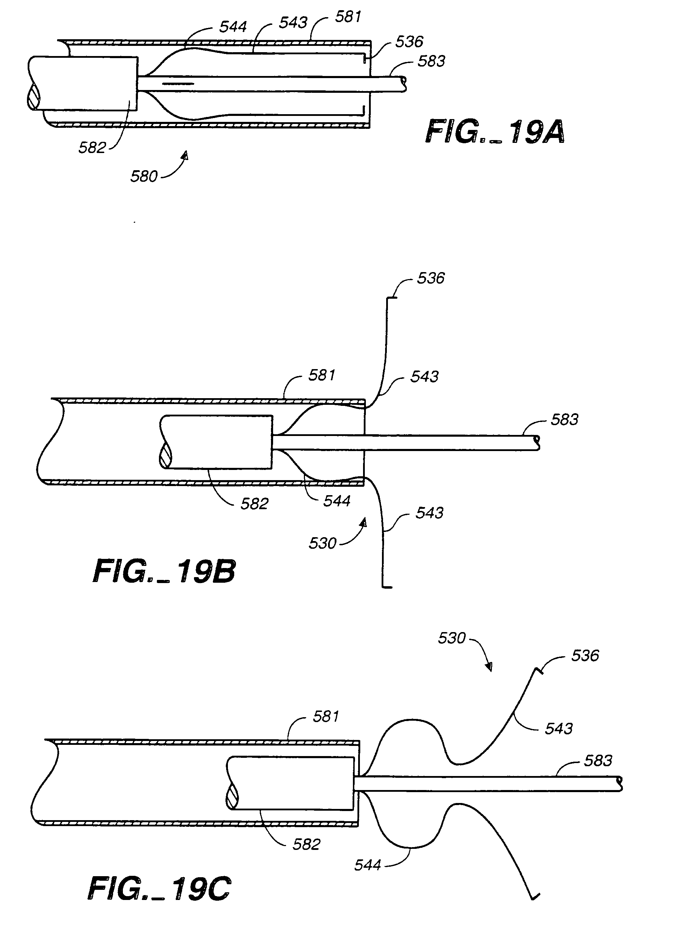

Figure 19A is a side view of a PFO closure device in accordance with the invention loaded into a catheter.

Figure 19B is a side view of the device of Figure 19A partially deployed out of the catheter.

Figure 19C is a side view of the device of Figure 19A fully deployed out of the catheter.

### Detailed Description:

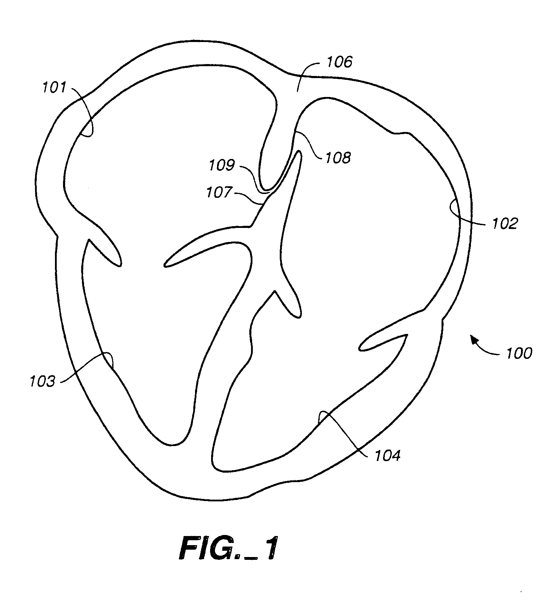

Referring to Figure 1, there is illustrated a heart 100 with a tissue defect, in this illustration, a patent foramen ovale 109. The heart 100 has a right atrium 101, right ventricle 103, left atrium 102 and left ventricle 104. The septum 106 between the right atrium 101 and the left atrium 102 comprises a septum primum 107 and a septum secundum 108. The patent foramen ovale 109 is an opening in the septum 106 that has not properly closed. Where a patent foramen ovale 109 is present, the septum primum 107 typically overlaps the septum secundum 108 and the higher pressure in the left atrium 102 typically closes the flaps of the septum primum 107 and the septum secundum 108 so that blood does not leak between the atria 101, 102. However, when there is a pressure change in the chest, the flaps may separate permitting blood to flow through the patent foramen ovale and between the atria 101, 102.

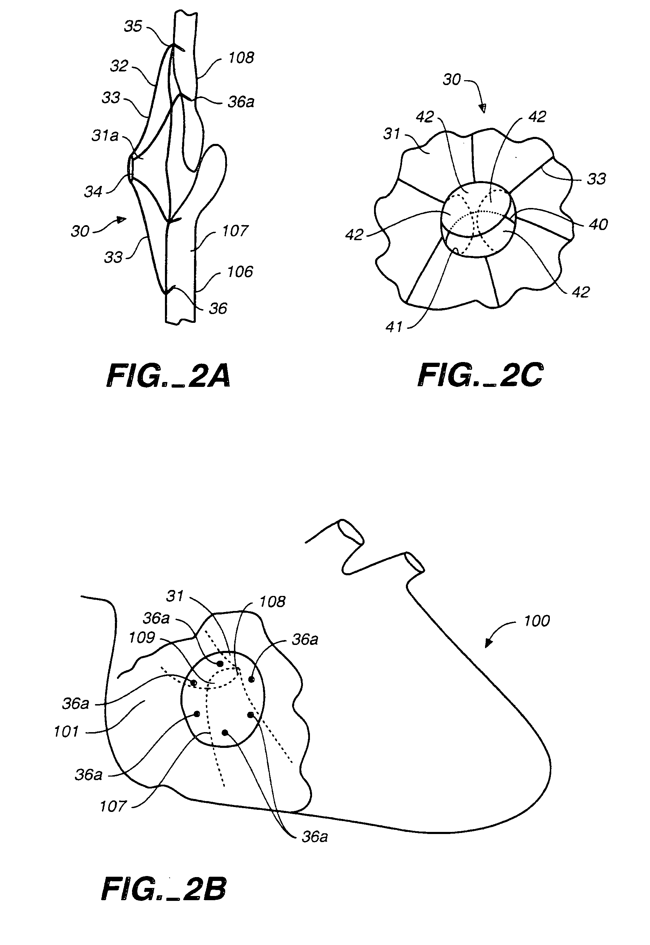

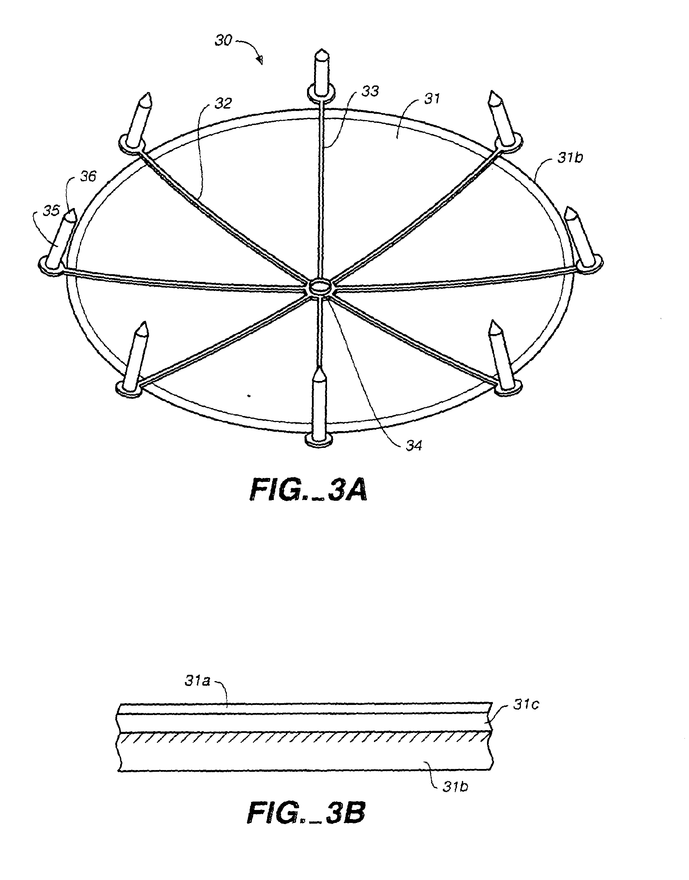

A PFO closure device 30 according to the invention is illustrated in Figures 2A-2C and 3A-3B. Figures 2A-2B illustrate the device 30 in place over the patent foramen ovale 109 in the right atrium 101 and attached to the septum 106. The device 30 is in the form of an umbrella-like structure or circular shaped patch 31 comprising a woven or mesh material such as, e.g., Dacron, ePTFE or metal. The patch 31 may have other shapes depending on the geometry of the anatomy of the PFO 109. For example, the patch 31 may be elliptical, arc shaped, split-shaped or otherwise customized based on a patient's anatomy. An expansion element 32 coupled to the patch 31, e.g., by stitching, may be used to spread, open or expand the patch from a compact configuration wherein the patch 31 may be delivered through a catheter, to an expanded or open configuration wherein the patch 31 may cover a portion of a wall of a heart chamber like an atrium wall. The patch covers a hole or other defect, including for example, a PFO 109.

As illustrated in Figures 2A and 3A, the expansion element 32 comprises a plurality of spokes 33 extending from a hub portion 34. The hub portion 34 includes an opening 41 therethrough for receiving a guidewire over which a delivery catheter and the patch device 30 are guided to the tissue defect site. As illustrated in Figure 2A, the distal side 40 of the patch device 30 has a plurality of petals 42 formed of the patch material that overlay each other to close the opening 41. When a guidewire is inserted through the opening 41 the petals 42 permit the guidewire to extend through the opening while when the guidewire is removed, the petals 42 close over the opening 41.

In addition, the device 30 comprises an attachment element 35 configured to engage the septum 106 from within the right atrium 101. The attachment element 35 as illustrated in Figures 3A and 3B is in the form of barbs 36 formed on or coupled (e.g. by welding) to the end of the spokes 33 and a surgical adhesive 31c may be applied to the outer rim 31b of the patch 31. (In one example, eyelets cut at the ends of the arms are fitted with spikes and fixed, to create barbs.) The surgical adhesive 31c is coated with a cap coat 31d of a dissolvable material such as a biocompatible bioresorbable material, for example, a glucose based absorbable material or polyglycolic acid or polylactic acid. The cap coat 31d is preferably selected and applied to dissolve at a predetermined rate so that the adhesive 31c is active after the device has been delivered via catheter to the surgical site and expanded. The surgical adhesive comprises a biocompatible adhesive that may be used in the circulatory system such as, for example, fibrin glue, BioGlue™, or FloSeal™.

The device 30 is shown deployed over the PFO 109 in Figures 2A and 2B. The patch 31 is opened by the expansion element 32 and the barbs 36 engage the septum primum 107 and the septum secundum 108 at points of engagement 36a. The cap coat 31d is exposed to body fluids in the circulatory system as it is being delivered and deployed whereby the cap coat dissolves at a predetermined rate and the glue becomes active to seal the patch 31 against the wall of the septum 106.

In variations of the invention either one or both of an adhesive and tissue engaging elements (such as barbs) may be used.

The device according to the invention further may have some surface modifications such as a textured or porous surface (open or closed pore) to promote tissue ingrowth; a coating or infusion of a material or substance that promotes a tissue response such as tissue ingrowth on or over the device to thereby further close any opening or patch any defect or that promotes tissue adhesion that improves the device's gripping of the heart or other structure. The tissue adjacent the device may also be treated, e.g., by ablating or otherwise causing tissue adhesions or scarring around the edges of the device to improve device fixation. For example a coating or surface treatment may be provided on the device's outer surface 31a to promote tissue growth closing around the PFO 109. Also the rim 31b of the device may be configured to promote tissue ingrowth or adherence to the device.

Furthermore, the device or any portion thereof in this or other examples herein may be constructed of a resorbable material so that it is absorbed or dissolved after a predetermined time in which the PFO has had an opportunity to close, or in which tissue has formed over the device or the opening. Such materials are generally known in the art and include materials such as, for example, silk keratin, collagen, gelatin, fibrinogen, elastin, actin, dextrin, chitin, and cellulose, as well as resorbable forms of polyolefin elastomers, silicones, block copolymers, filled polymers, and hydrogels. The materials may be selected for their specific resorption properties such as resorption rate.

Additional features of the device and its delivery are illustrated in Figures 4-7.

Figures 4-7 illustrate a method and device for delivering a PFO closure device 30 in accordance with an embodiment of the invention. Referring to Figure 4, the device 30 is in a compressed configuration within a catheter 80. A delivery device 81 comprises an inner member push tube 82 extending through the catheter 80 to a push rod engaging element 37 on the device 30. The inner member push tube 82 includes a lumen therethrough for receiving a guidewire 84. A middle sheath 83 is slidably positioned over the inner member push tube 82 and expanders 85 comprising miniature hypotubes are coupled to the middle sheath 83. The middle sheath 83 is actuable to extend and retract the expanders 85 coupled to the middle sheath 83.

In use, a guidewire 84 is inserted through a patient's vasculature, through the vena cava into the right atrium 101 of the heart 100. The guidewire 84 is then passed through the patent foramen ovale 109. The catheter 80 containing the delivery device 81 and PFO closure device 30 is then passed over the guidewire 84 into the right atrium 101, locating the catheter and the device 30 adjacent the PFO 109. The delivery device 81 push tube 82 engages the push element 37 of the PFO closure device 30 while expanders 85 of the delivery device 81 are removably coupled to the device 30.

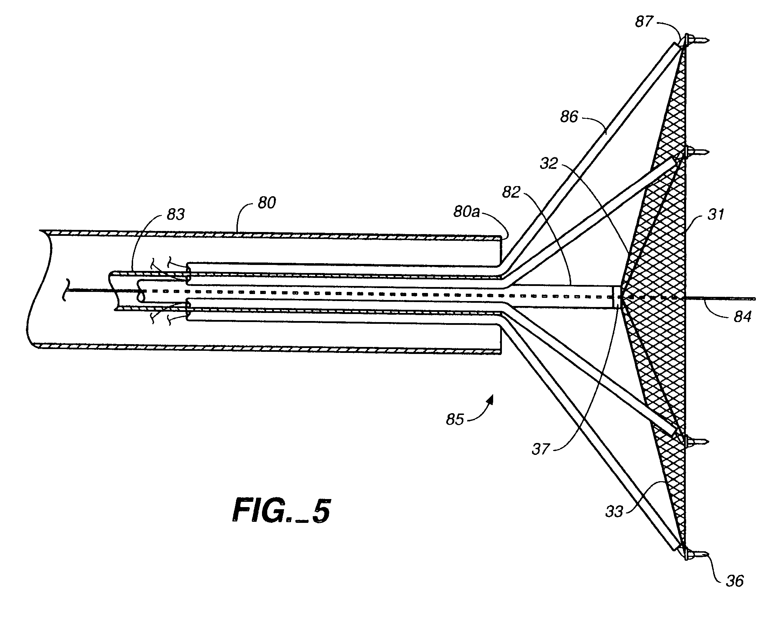

As shown in Figure 5, the inner push tube 82 is advanced, engaging the push element 37 of the device 30 which pushes the PFO closure device 30 out of the distal end 80a of the catheter 80. The spokes 33 of the expansion element 32 are constructed of a spring material, e.g., of a shape memory alloy such as Nitinol. The spokes 33 tend to expand to an open position such as that illustrated in Figure 5, opening the patch 31.

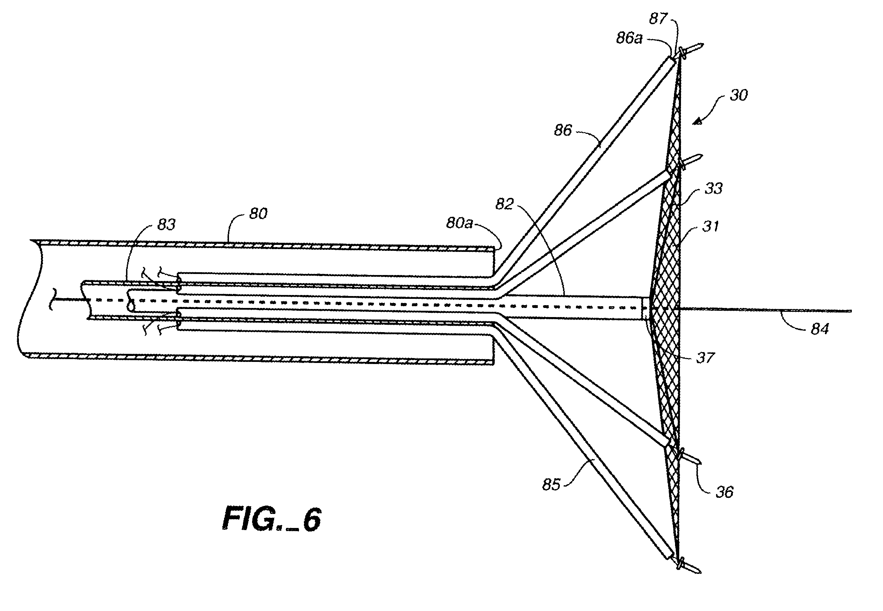

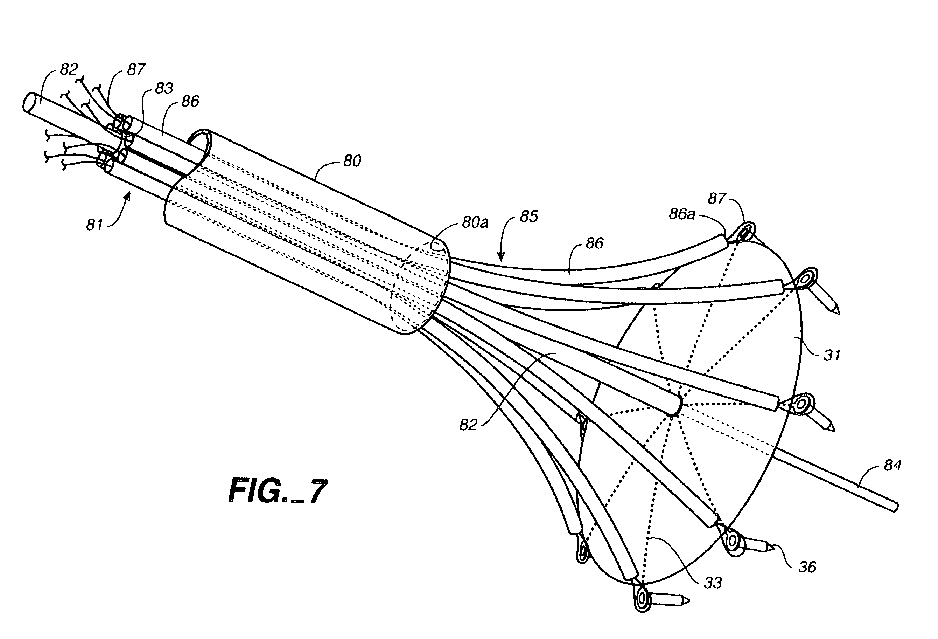

The expanders 85 illustrated in Figures 4-7 comprise a plurality of miniature flexible hypotubes 86 opening adjacent the barbs 36 on the device 30. Each of the hypotubes 86 have sutures 87 extending through the catheter 80 into the hypotube 86 and then out of the distal end 86a of the hypotube 86 and looping around a corresponding barb 36. The sutures 87 may be actuated through the catheter 80 by pulling on the sutures 87 to draw the patch 31 back, i.e., further open it into a hyper-extended position (Figure 6). The sutures 87 and hypotubes 86 may also be used to stabilize the patch device 30 while the push tube 82 is extended to further open or hyper-extend the device 30 during deployment. The combination of positioning the hypotubes 86 against the device, pulling the sutures 87 and pushing the device 30 with the push tube 82 pulls the barbs 33 back into a position where they are generally perpendicular to the wall of the septum 106 as shown in Figure 6. The device 30 may then be advanced into position over the PFO 109 with the barbs 33 piercing or engaging the septal wall (i.e., both the septum primum 107 and the septum secundum 108). When the sutures 87 are released and removed and/or the push tube 82 is released from the device 30, the device 30 will tend to move back to its relaxed position as illustrated in Figures 5 and 7. The barbs 33 then hook into the wall of the septum 106 as illustrated in Figure 2A.

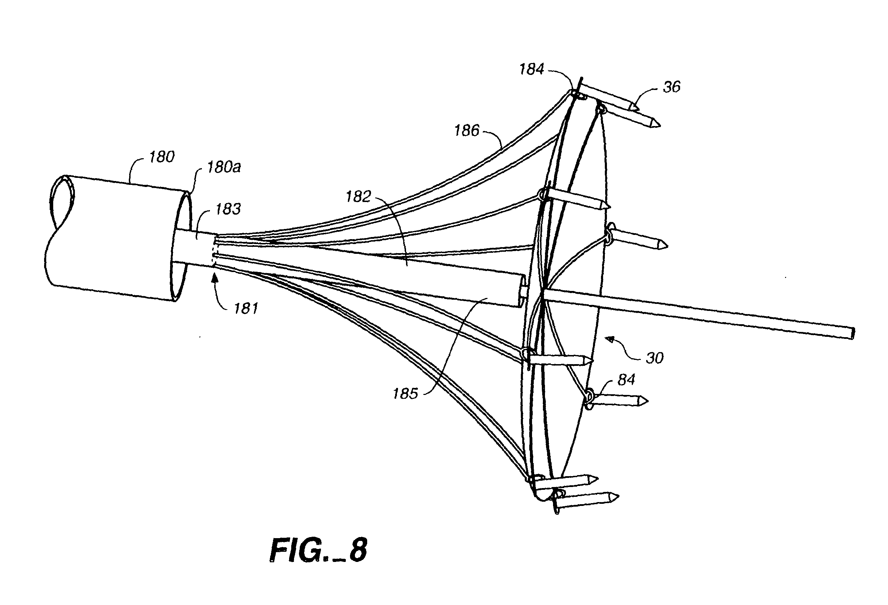

Figure 8 illustrates an alternative deployment device 181. The deployment device 181 is shown in Figure 8 extending out of the distal end 180a of the catheter 180. The deployment device 181 comprises flexible bars 186 coupled to a middle sheath 183, and each having a hook 184 located on the distal end of a bar 186. Each hook 184 is hooked around a barb 36 of the PFO closure device 30. The bars 183 are retracted to stabilize and extend the device 30 as it is deployed while a push rod 182, slidable within the middle sheath 183, stabilizes and hyper extends the device 30. After deployment the middle sheath 183 is twisted, releasing the hooks 184 from the device 30 so that the catheter 180 and deployment device 181 may be removed.

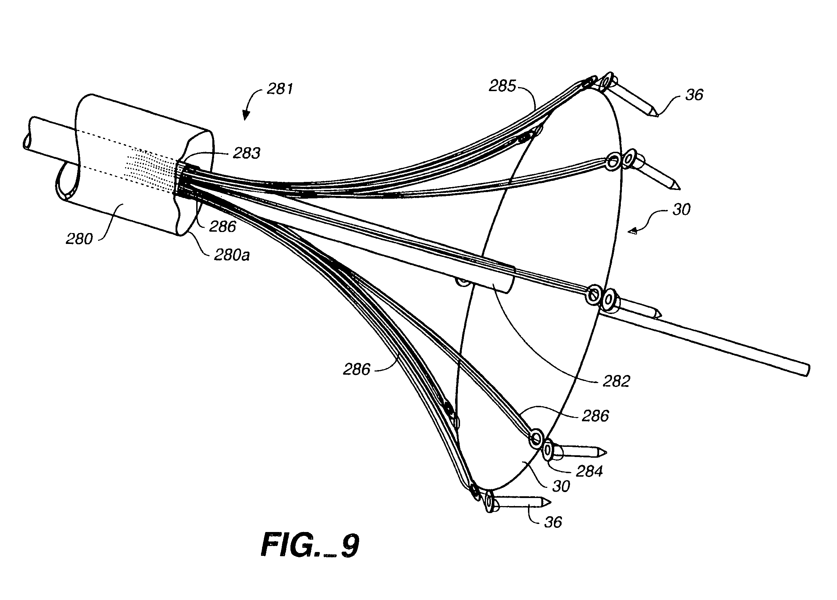

Figure 9 illustrates an alternative deployment device 281. The deployment device 281 is shown in Figure 9 extending out of the distal end 280a of the catheter 280. The deployment device 281 comprises flexible bars 286 coupled to a middle sheath 283, each having an eyelet 284 on its distal end 281a for threading one of a plurality of sutures 285. Each of the plurality of sutures 285 is further looped around or coupled to a barb 36 of the PFO closure device 30. The sutures 285 then extend proximally through the catheter 280 so they may be pulled to retract the spokes 34 and barbs 36 of the PFO closure device and/or to stabilize the device 30 while the push rod 282 slidable within the middle sheath 283 hyper-extends and/or stabilizes the device 30 as it is deployed. After deployment the sutures 285 are cut from the proximal end so that one cut end of each of the sutures can be pulled to release the sutures from the device 30 so that the catheter 280 and deployment device 281 may be removed. The sutures 285 may each comprise a suture loop that may be cut and the pulled out of the catheter 280 from its proximal end.

Referring to Figures 10 and 11, the PFO closure device 30 is illustrated having slightly different initial relaxed configuration (Figure 10) and a slightly different hyper-extended configuration (Figure 11) than illustrated in Figure 1-7. These configurations may be set in the material of the extension member 32 of the device 30 and/or the patch 31. As illustrated in Figure 10, the relaxed position is flat so that when the device 30 is deployed, it tends towards a flat position against a septum 106. Likewise, when the device 30 is hyper-extended as illustrated in Figure 11, it is convex with respect to its interface with the septal wall so that the barbs 36 may be positioned to be placed in the septal wall and then may return to the position illustrated in Figure 10 after deployment so that the barbs 36 grab the tissue of the septum 106.

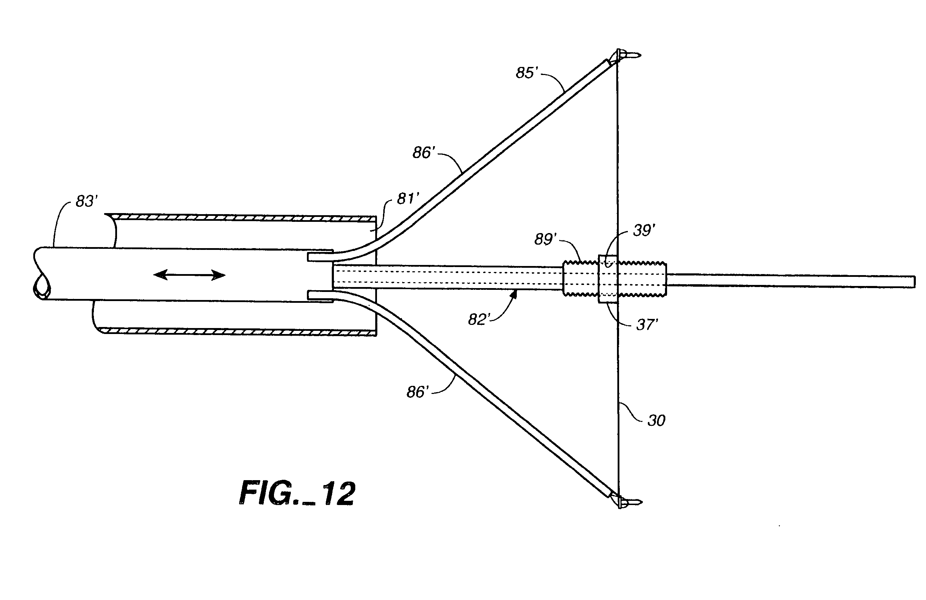

Figure 12 illustrates the delivery device 81' similar to delivery device 81 of Figures 4-7, with an alternative push rod 82' and the PFO closure device 30 with an alternative push rod engaging element 37'. The push rod 82' has a threaded (or alternatively keyed) end 89' that screws (or otherwise connects) into threaded (or keyed) opening 39' of the push rod engaging element 37'. The PFO closure device 30 is deployed as described with reference to Figures 4-7 except that the push rod engaging element 37' and the push rod 82' are engaged by threading the two together. The expanders 85' comprising flexible hypotubes 86' coupled to middle sheath 83' contain sutures similar to hypotubes 86 described above with reference to Figures 4-7, hold the device 30 in place deployed over the PFO while the push rod 82' is unscrewed from the push rod engaging element 37'. The expanders 85 are then retracted as described herein with reference to Figures 4-7. The device 30 may be retrieved after deployment by coupling the end89' to the push rod engaging element 37' and retracting the device 30 into the catheter 80.

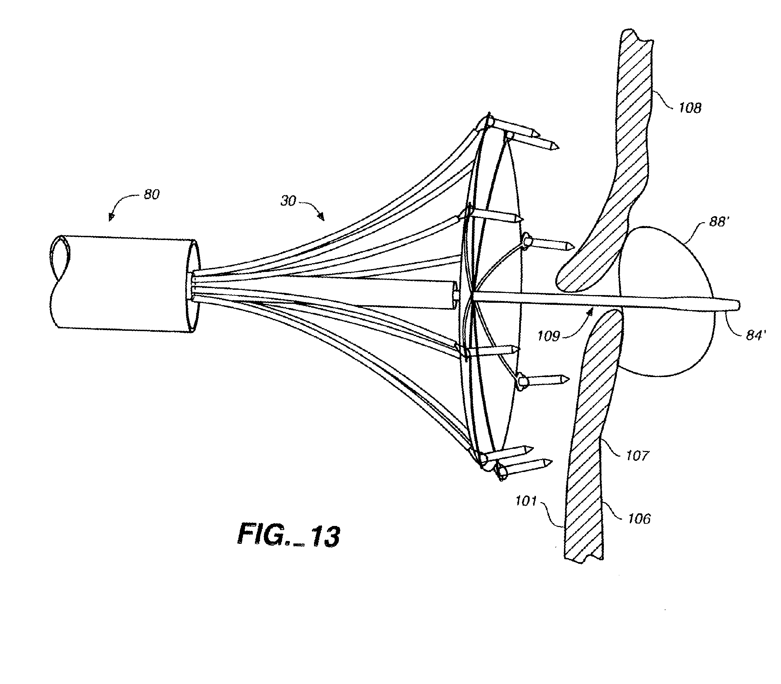

Figure 13 illustrates a variation of the delivery system of Figures 4-7. The PFO closure device 30 is illustrated being deployed through the catheter 80 over the PFO 109. According to this variation, instead of a guidewire guiding the catheter 80 and device 30 towards the PFO 109, a balloon catheter 84' is place through the PFO 109. A balloon 88' at the tip of the balloon catheter 84' is inflated to expand on the opposite side of the PFO 109 (the left atrium 102). The push rod 82 (Figures 4-7) pushes the device 30 into place while the balloon catheter 84' and balloon 88' stabilized and direct the delivery device 81 (Figures 4-7) as it deploys the device 30. The balloon 88' and the device 30 are aligned with respect to the same guidewire, accordingly, the balloon 88' may also act to center the device 30 over the PFO. The device delivery system is thus self-centering. The balloon 88' is deflated when the PFO device 30 is in place, and the balloon catheter 84' is retracted into the catheter 80. Other know centering devices may be used as well.

Figures 14A-14C illustrate another embodiment of a PFO closure device 330 according to the invention. The device 330 comprises a tubular portion 331 constructed of a material such as Dacron or ePTFE and annular support members 332 coupled to the tubular portion (e.g., by stitching). The annular members 332 may be spring members formed of a material such as Nitinol preset to a first configuration as illustrated in Figure 14A. A plurality of barbs 335 is located on the distal end 336 of the device 330 to engage the tissue of the septum 106. The top of the device 333 is covered with a resilient elastic material such as biodegradable and biostable elastomers, polyolefin elastomers, silicones (homo and co-polmers), block co-polymers (e.g., polyurethane, and polyurethane blends with PTFE); filled polymers (e.g., with hydroxyapatite fillers), hydrogels, collagen, elastin, polyesters acrylics, shape memory polymer. These materials may also be surface modified, either chemically or physically, to render them non-thrombogenic. The top of the device 333 has a slit 334 through the material for passing a balloon catheter or other device that may be used to expand or open the device 330 to an open configuration such as illustrated in Figure 14B. The slit 334 will close when the catheter or expansion device is removed as illustrated in Figure 14C. The PFO closure device 330 may be expanded to an open position whereby the sharp elbow portion 337 of the barb 335 is forced into the tissue of the septum 106. Thus, the device 330 is deployed over the PFO 109 (see Figure 17). The natural recoil of the device 330 back to its preset configuration (see Figure 14C) draws the ends 338 of barbs 335 into engagement with the tissue of the septum 106 and holds the septum primum 107 and the septum secundum 108 together. A substance promoting tissue growth may be coated over the device to encourage tissue growth over the device 330 and between the septum primum 107 and the septum secundum 108 after a period of time. Tissue growth may also occur at the wall of the left atrium 102 of the heart 100 as the device 330 holds the septum primum 107 and septum secundum 108 in place for a sufficient duration to permit such growth.

As shown in Figure 15, the device 330 is being deployed. A guidewire 340 has been placed through the vasculature, into the right atrium 101, through the PFO 109 and into the left atrium 102. The device 330 is preloaded on a balloon catheter 350. The balloon catheter tip 351 is placed through the slit 334 and the annular members 332 are loaded over the balloon catheter tip 351 in a compressed position in a similar manner to how an expandable stent is loaded on a balloon catheter. The balloon catheter 350 is guided over the guidewire 340 to a position within the right atrium 101, the balloon tip 351 of the catheter 350 and the barbs 335 of the device 330 positioned adjacent the PFO 109. The balloon catheter tip 351 is expanded, expanding the device 330 from its first position as illustrated in Figure 14A, to a second expanded position as illustrated in Figure 14B. The elbows 336 of the barbs 335 pierce the wall of the septum primum 107 and the septum secundum 108 upon expansion of the balloon catheter tip 351. Upon deflation of the balloon catheter tip 351, the recoil of the device 330 tends to draw the barbs 335 in towards the device 330 so that the ends 337 of the barbs 335 engage the tissue of the septum 106 (Figures 16 and 17).

Figures 18A and 18B illustrate the PFO closure device 30 and a delivery device 441 according to the invention. The delivery device 441 is positioned within a catheter 440 that has accessed the deployment site through the vasculature. The delivery device 441 includes a stent push member 443 that engages and pushes the device 30 during deployment. The stent push member 443 is configured like a stent with a series of attached diamond shaped structures or annular support members that provide columnar strength while being collapsible to a smaller diameter when retracted. The push stent member 443 may be constructed of a shape memory alloy that is preset to a predetermined diameter. When the push stent member 443 is advanced out of the catheter 440 that is holding it in a compressed position, it expands to its preset shape. The stent member may also include sutures tied around the stent and extend to the proximal end of the catheter whereby the sutures may be pulled to draw the stent member into a smaller diameter shape when it is retracted. The push member 443 is attached, e.g., by heat welding, to a middle sheath 442, which is positioned over the proximal end of the push stent 443. An inner sheath 444 is positioned within the push member 443. The inner sheath 444 includes an engaging tip 450 that engages the push rod engaging element 37 of the device 30 to additionally push and maneuver the device 30. A pull device 449 is positioned in the sheath 444 and is configured to pull or stabilized the patch device 30 against the push stent device as it is being deployed. The pull device 449 includes a first wire 445 attached to the patch device 30 and ball 446 attached to the wire 445. The pull device 449 further includes a second wire 447 extending out of the proximal end of the catheter, and a ball 448 attached to the wire 446. The balls 446, 448 are positioned in an engaging position with each other, with each ball positioned on the opposite side of the other ball from the direction to which their respective wires 445, 447 lead. Thus in a pulling relationship, the balls 446, 448 engage one another. The sheath 444 is placed over the balls 445, 447 and wires 446 and extends out of the proximal end of the catheter where it may be pushed to engage the device 30 or retracted to release the balls 446, 448 from engaging one another.

In use, the PFO closure device 30 is loaded in the catheter 440 with the device 30 and the push stent 443 compressed to a low profile. The pull device 449 is coupled to the patch 30 with balls 445, 447 of the pull device 449 creating an engaged relationship between the PFO closure device 30 and the pull device 449 that allows the pull device 449 to exert a pull force on the PFO closure device 30. The sheath 442 and inner member 444 are advanced out of the catheter 440, the inner member 444 pushing the push rod engaging element 37 of the device 30. The push stent 443, which is attached to the middle sheath 442, is thereby advanced out of the catheter 440. As the push stent 443 is advanced out of the catheter 440, it expands to a preset shape having an outer diameter to match the outer diameter of the device 30. Thus, the push stent 443 and the inner sheath 444 push and maneuver the device 30 while the pull device 449 stabilizes it. The inner member 444 may be used to push and hyper-extend the patch 31 so that the barbs 36 are generally perpendicular to the tissue as the patch is deployed. The patch 31 is positioned over a PFO or other defect and is pushed into the tissue so that the barbs 36 engage the tissue and the adhesive further attaches the device to the tissue. The sheath 444 is then retracted into the catheter 440 while the wire 447 is released. The sheath 444 that held the balls 446, 448 in an engaged position is thereby removed permitting disengagement of the ball 448 and wire 447 from ball 446 and wire 445, releasing the device. The ball 448 and wire 447 are also retracted into the catheter 440 as well as the push member retracted by the middle sheath 442 to which it is attached.

Figures 19A-19C illustrate a patch device 530 and delivery system 580 in accordance with the invention. The patch device 530 includes a patch material similar to the patch material 31 described above with reference to Figures 4-7. A plurality of spokes 543 formed of Nitinol or a spring material are attached to the patch material, also in a similar manner as the spokes and patch material described with reference to Figures 4-7. A plurality of barbs 536 are formed at the distal ends of the spokes 543. The spokes 543 join at the proximal end of the patch device 530 to form a bulb shaped proximal portion 544 when the spokes 543 are in their preset shape. When in this position, the barbs 536 tend to point inward so that they will engage tissue when the patch device 530 is deployed in tissue. The bulb shaped proximal portion 544 may be deflected to draw the spoke 543 outward as illustrated in Figure 19B. In this position the barbs 536 are generally pointing in the direction of the catheter 581 of the delivery system 580 and thus generally perpendicular to a wall of tissue into which the device 530 is directed by the catheter 581.

The device 530 is initially positioned within the catheter 581 in a compressed configuration wherein the bulb shaped proximal portion 544 is deflected while the spokes 543 are contained within the catheter 581 in a closed position. A guidewire 584 is positioned through the catheter 581, through the patch device 530, and into a PFO (not shown) to guide the patch device 530 into position. A push rod 582 is positioned within the catheter 581, proximally of the patch device 530. As illustrated in Figure 19B, the push rod 582 is advanced predetermined distance to a first stage where the spokes 543 extend out of the catheter 581 and the catheter distal end 585 contains and depresses the bulb shaped proximal end 544 of the device 530. With the barbs 536 extending straight out, the device 530 is directed into the tissue with the catheter 581. As illustrated in Figure 19C, the push rod 582 is then advanced so that the device is deployed out of the catheter 581. When released from the catheter 581, the bulb shaped proximal end 544 returns to is preset bulb shaped configuration whereby the barbs 536 turn inward to engage the tissue.

While the invention has been described with reference to particular embodiments, it will be understood to one skilled in the art that variations and modifications may be made in form and detail without departing from the spirit and scope of the invention. For example, the device may be used to patch a variety of defects or holes in various locations within the circulatory system including the heart and vasculature. Means for attaching the patch to the wall of the heart may include other similar means such as glues, bonds, and mechanical means such as, for example, staples, clips, or sutures.

## Claims

1. A tissue defect patch device for patching a heart tissue defect the patch device comprising:
a patch material configured to cover a portion of a side of a heart tissue defect; and
a tissue attacher coupled to the patch device and configured to attach the patch device to tissue on said side of the tissue defect.

2. The tissue defect patch device of claim 1 wherein the patch material comprises a distal side defining a first distal surface and a proximal side defining a second proximal surface, wherein the patch device is configured to cover a portion of a side of a tissue defect.

3. The tissue defect patch device of claim 1 or 2 wherein the tissue attacher comprises a barb.

4. The tissue defect patch device of claim 1 or 2 wherein the tissue attacher comprises an adhesive.

5. The tissue defect patch device of any of claims 1 to 4 further comprising:
an expander coupled to the patch device and configured to expand the patch device from a first position to a second open position.

6. The tissue defect patch device of claim 5 wherein the patch device is configured to engage the tissue in the second open position; wherein the expander is releasable; and wherein when the expander is released, the patch device tends to move towards the first position to thereby attach the device to said side of the tissue defect.

7. The tissue defect patch device of claim 5 wherein the expander comprises a deflectable member where when deflected, the patch device moves to the second open position and where the deflectable member is released, the patch device moves to the first position.

8. The tissue defect patch device of any of claims 5 to 7 wherein the expander comprises a plurality of prongs extending from a central portion of the patch device.

9. The tissue defect patch device of claim 8 wherein the prongs comprise a spring material.

10. The tissue defect patch device of any of claims 5 to 9 wherein the expander comprises an annular member.

11. The tissue defect patch device of any of claims 1 to 10 further comprising a delivery device coupling element configured to couple with a delivery device to position the patch device.

12. The tissue defect patch device of claim 11 wherein the delivery device coupling element comprises a releasable attachment element to releasably attach the patch device to the delivery device.

13. The tissue defect patch device of any of claims 1 to 12 wherein the device comprises a septal closure device configured to patch a defect in the septal wall of a heart.

14. A tissue defect patch device for patching a heart tissue defect, the tissue defect patch device comprising:
means for patching a heart tissue defect on one side of the defect; and
means for attaching the means for patching to the one side of the tissue defect without passing through the defect.

15. A tissue defect patch device for patching cardiovascular tissue comprising:
a patch material comprising a distal side defining a first distal surface and a proximal side defining a second proximal surface the patch material being configured to cover a portion of a side of a tissue defect; and
a tissue attacher coupled to the patch device and configured to attach the patch device to tissue on said side of the tissue defect.

16. A method for patching a heart tissue defect comprising the steps of:
providing a patch device;
delivering the patch device adjacent a heart tissue defect on one side of the heart tissue defect; and
attaching the patch to tissue on the one side of the defect.

17. The method of claim 16 wherein the step of providing a patch device comprises providing a patch device having a first position expandable to a second open position, and providing an attachment mechanism coupled to the patch device, further comprising the steps of:
expanding the patch device to the second open position; and
engaging the attachment mechanism with tissue on said side of the defect.

18. The method of claim 17 wherein the step of engaging the attachment mechanism comprises piercing the heart tissue with the attachment mechanism.

19. The method of claim 18 wherein the attachment mechanism comprises at least one barb and wherein the step of piercing the heart tissue with the attachment mechanism comprises piercing the heart tissue with the at least one barb.

20. The method of claim 17 wherein the step of providing a patch device comprises providing a patch device having a spring mechanism biasing the device from the second position towards the first position, further comprising the step of releasing the patch device from the second position after engaging the attachment device with the septal wall whereby the attachment device attaches to the septal wall.

21. The method of claim 17 further comprising the steps of:
providing a deployment device comprising at least one expander configured to move the patch device from the first position to the second open position, and initially providing the patch device coupled to the at least one expander of the deployment device; and
wherein the step of expanding the patch device comprises expanding the patch device with the at least one expander.

22. The method of claim 16 further comprising the steps of:
providing a catheter having a distal portion and containing the patch device and the deployment device;
positioning the distal portion of the catheter in the right atrium of a heart adjacent a defect in a septal wall;
advancing the patch device out of the catheter distal end to deploy the patch device.

23. The method of claim 16 wherein the step of attaching the patch to tissue comprises attaching the patch to tissue with an adhesive.

24. A system for patching a defect in heart tissue comprising:
a patch device comprising:
a patch material configured to cover a portion of a side of a heart tissue defect; and
a tissue attacher coupled to the patch device and configured to attach the patch device to tissue on said side of the tissue defect; and
a deployment device comprising:
a catheter containing the patch device; and
a push member positioned in the catheter adjacent the patch device and configured to push the patch device out of the catheter.

25. The system of claim 24 wherein the deployment device contains a pulling member removably coupled to the patch device.

26. The system of claim 24 or 25 wherein the deployment device is removably and reattachably coupled to the patch device.

27. The system for patching a defect in heart tissue of any of claims 24 to 26 further comprising:
an expander coupled to the patch device and configured to expand the patch device from a first position to a second open position; and wherein the deployment device comprises:
an expansion element coupled to the expander of the patch device and configured to move the patch device from the first position to the second open position.

28. The system for patching a defect in heart tissue of claim 27 wherein the patch device is configured to engage the tissue in the second open position; wherein the expander is releasable; and wherein when the expander is released, the patch device tends to move towards the first position to thereby attach to said side of defect.

29. The system for patching a defect in heart tissue of claim 27 or 28 wherein the expander comprises a plurality of prongs extending from a central portion of the patch device.

30. The system for patching a defect in heart tissue of any of claims 27 to 29 wherein the expander comprises an annular member.

31. The system for patching a defect in heart tissue of any of claims 24 to 30 wherein the patch device comprises a septal closure device configured to patch a defect in the septal wall of a heart.
